# FASCICULE DE BREVET EUROPEEN

(11) **EP 1 889 642 B1**
(45) Date de publication et mention de la délivrance du brevet: **08.05.2019**
(21) Numéro de dépôt: 07301201.5
(22) Date de dépôt: 02.07.2007
(51) Int. Cl.: A61K 8/44, A61K 8/60, A61Q 19/08

(54) **Compositions cosmétiques associant un dérivé C-glycoside et un dérivé N-acylaminoamide**
Kosmetische Zusammensetzungen vereinend ein C-Glykosid-Derivat und ein N-Acylaminoamid-Derivat
Cosmetic compositions combining a C-glycoside derivative and a N-acylaminoamide derivative

(30) Priorité: 03.07.2006 FR 0606014
(43) Date de publication de la demande: 20.02.2008
(73) Titulaire: L'Oréal, 75008 Paris (FR)
(72) Inventeur: Pineau, Nathalie, 95220, Herblay (FR); Catroux, Philippe, 33230, Guitres (FR)
(74) Mandataire: Nony

(56) Documents cités:
- EP-A- 1 589 010
- DE-A1- 10 341 234
- FR-A1- 2 810 033
- ZA-A- 200 303 674

## Description

La présente invention se rapporte au domaine des compositions cosmétiques et/ou dermatologiques.

Plus particulièrement, elle vise à proposer de nouvelles compositions cosmétiques et/ou dermatologiques comprenant une association entre un composé inhibiteur de l'élastase de la famille des N-acylaminoamides et au moins un activateur de la synthèse des glycosaminoglycannes et de collagène VII, choisi parmi des dérivés C-glycosides.

Ces compositions s'avèrent particulièrement avantageuses pour prévenir et/ou traiter les signes cutanés du vieillissement et/ou du photovieillissement et en particulier augmenter l'épaisseur épidermique en augmentant la différenciation kératinocytaire.

L'épiderme est un épithélium pavimenteux stratifié kératinisé. Son épaisseur moyenne varie de 60 à 100 µm et peut atteindre 600 à 700 µm à la plante des pieds et à la paume des mains. Il est constitué principalement de kératinocytes (90 %), mais également d'autres cellules, et repose sur une membrane basale qui le sépare du derme.

La différenciation progressive des cellules de la membrane basale vers la surface de l'épiderme s'accompagne d'une multitude de changements. A titre d'exemple, il faut citer la kératinisation, soit l'expression, la synthèse, l'organisation des diverses kératines, leurs interactions avec d'autres protéines et leurs modifications tout au long de la différenciation.

La différenciation, processus programmé génétiquement, s'observe en microscopie par des changements structuraux observés au sein des diverses couches épidermiques qui, à partir de la membrane basale, se nomment : basale, épineuse, granuleuse et cornée. En se différenciant, la cellule devient de plus en plus spécialisée et limitée dans ses structures et ses fonctions.
- Ainsi, les kératinocytes de la couche basale, liés à la jonction dermo-épidermique par des hémidesmosomes, sont cubiques ou prismatiques et ils s'unissent entre eux par des desmosomes.
- Abondants dans la couche épineuse, les desmosomes donnent un aspect épineux aux kératinocytes. Ceux-ci s'aplatissent et augmentent de volume dans la couche épineuse ainsi que dans la couche granuleuse. L'apparition des granules de kératohyaline confère le nom à cette couche dont les kératinocytes possèdent également des corps lamellaires, des organelles encore en métabolisme actif et des desmosomes.

- Lors de la transition de la couche granuleuse à la couche cornée, les organelles disparaissent, les granules de kératohyaline s'étendent en de grandes masses dans le cytoplasme, les corps lamellaires se déversent dans l'espace intercellulaire élargi, les macrofibrilles de kératine s'accumulent et la membrane cytoplasmique s'épaissit.
- Les cornéocytes sont des résidus cellulaires inertes dotés d'un robuste cytosquelette de kératine, d'une enveloppe cornée protectrice sous la membrane cytoplasmique enrobée de lipides intercellulaires.
- Au terme de la différenciation, la cellule est devenue un amas de kératines pratiquement inertes.

Cette propriété de kératinisation qu'ont les kératinocytes, associée à celle d'être des cellules très jointives grâce à leurs nombreuses jonctions cellulaires, procurent à l'épiderme une résistance accrue aux agressions de l'environnement. Les cellules de la couche cornée, certes mortes mais protectrices, desquament et sont remplacées par d'autres grâce à la prolifération des cellules basales de telle sorte que la perte et la multiplication cellulaires soient bien équilibrées.

Lors de la ménopause, la peau subit des modifications dans tous ses compartiments, à savoir dermiques et épidermiques.

Les principales modifications concernant le derme sont une diminution du taux de collagène et de l'épaisseur dermique. Cela entraîne chez la femme ménopausée un amincissement de la peau et/ou des muqueuses. La femme ressent alors une sensation de "peau sèche" ou de peau qui tire et l'on constate une accentuation des fines rides et ridules de surface. La peau présente un aspect rugueux à la palpation. Enfin, la peau présente une souplesse diminuée.

Les principales modifications concernant l'épiderme sont une diminution de la différenciation des kératinocytes entraînant un déficit de la matrice protéique de la cellule cornée, une augmentation des métalloprotéinases, protéases dégradant la matrice extracellulaire et qui participent au vieillissement cutané, ainsi qu'à une diminution de la synthèse des différents glycosaminoglycannes.

La présente invention a précisément pour objectif de proposer une solution à ces différents problèmes, et notamment de proposer de nouvelles compositions susceptibles d'être utilisés dans les domaines cosmétique et pharmaceutique pour limiter le vieillissement de la peau, qu'il soit chronobiologique ou photoinduit, et notamment le vieillissement généré par une diminution de l'élasticité de la peau et/ou par une dégradation du collagène dans la structure des tissus et/ou par une diminution de la synthèse des glycosaminoglycannes.

Le brevet EP 1 292 608 décrit l'efficacité à ce titre de dérivés N-acylaminoamide. Plus précisément, ces dérivés s'avèrent notamment intéressants au regard de leur aptitude à ralentir l'activité de dégradation des fibres élastiques des espaces intercellulaires.

De même, les sucres et dérivés du sucre sont des produits déjà mis à profit à des fins diverses pour la formulation de compositions cosmétiques destinées tant au soin de la peau qu'au soin et/ou lavage des fibres kératiniques. Ainsi, dans WO 99/24009, le D-xylose et ses dérivés sont proposés à des fins de préparation de produits cosmétiques ou pharmaceutiques visant à améliorer la fonctionnalité des cellules de l'épiderme.

Le document DE 103 41234 concerne de nouveaux dérivés N-acyl-hydroxyamino-acide alkylamide de structure de type céramide et leur utilisation dans des compositions pour la protection de la peau ou des cheveux.

Certains dérivés C-glycosides ont démontré des propriétés biologiques intéressantes, en particulier pour activer la synthèse des glycosaminoglycannes et de collagène VII. De tels composés sont notamment décrits dans le document WO 02/051828.

Ces composés sont plus particulièrement représentés par la formule : 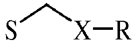 dans laquelle S représente un monosaccharide ou un polysaccharide, R représente différents radicaux linéaires ou cycliques et le groupement X peut représenter un groupement choisi parmi : -CO-, -CH(NR₁R₂)-, CHR'-, -C(=CHR')- avec R' pouvant représenter différent radicaux, dont le radical hydroxyle.
Le document EP 1 589 010 concerne un procédé de synthèse de nouveaux dérivés C-glycosides et leur utilisation pour lutter contre le vieillissement de la peau.

De façon surprenante, les inventeurs ont constaté qu'un composé de la famille des N-acylaminoamide, en association avec un dérivé C-glycoside présente une activité synergique pour stimuler la différenciation des kératinocytes et donc augmenter l'épaisseur et la qualité de l'épiderme.

En conséquence, un premier aspect de l'invention concerne une composition cosmétique et/ou dermatologique selon la revendication 1 associant dans un milieu physiologiquement acceptable au moins un dérivé N-acylaminoamide à au moins un dérivé C-glycoside.

L'invention concerne, selon un autre de ses aspects, l'utilisation cosmétique d'au moins un dérivé N-acylaminoamide en association avec au moins un dérivé C-glycoside tels que définis en revendication 1, ou d'une composition de l'invention, pour traiter, de manière préventive ou curative, les signes du vieillissement de la peau du corps ou du visage.

L'invention concerne encore, selon un autre de ses aspects, l'utilisation cosmétique d'au moins un dérivé N-acylaminoamide en association avec au moins un dérivé C-glycoside tels que définis en revendication 1, ou d'une composition de l'invention, pour traiter, de manière préventive ou curative, le manque d'élasticité et/ou de tonus de la peau.

Il est aussi décrit l'utilisation, notamment cosmétique, d'au moins un dérivé N-acylaminoamide en association avec au moins un dérivé C-glycoside ou d'une composition telle que définie ci-dessus, pour traiter, de manière préventive ou curative, les signes du vieillissement de la peau du corps ou du visage, qu'ils soient chronobiologiques ou photo-induits, et notamment le vieillissement généré par une diminution de l'élasticité de la peau et/ou par une dégradation du collagène dans la structure des tissus.

Il est encore décrit l'utilisation, notamment cosmétique, d'au moins un dérivé N-acylaminoamide en association avec au moins un dérivé C-glycoside ou d'une composition telle que définie ci-dessus, pour traiter, de manière préventive ou curative, les rides et/ou ridules, la peau flétrie, le manque d'élasticité et/ou de tonus de la peau, l'amincissement du derme, la dégradation des fibres de collagène, la peau molle, la peau amincie, les dégradations internes de la peau consécutives à une exposition aux rayonnements ultra-violets.

Il est encore décrit l'utilisation, notamment cosmétique, d'au moins un dérivé N-acylaminoamide en association avec au moins un dérivé C-glycoside ou d'une composition telle que définie ci-dessus, pour inhiber l'activité des élastases et/ou pour limiter et/ou combattre la dégradation des fibres élastiques.

Il est encore décrit l'utilisation, notamment cosmétique, d'au moins un dérivé N-acylaminoamide en association avec au moins un dérivé C-glycoside ou d'une composition telle que définie ci-dessus, pour augmenter la différenciation des kératinocytes.

De manière inattendue, il a été montré dans le cadre de la présente invention qu'une association d'au moins un dérivé C-glycoside avec au moins un dérivé N-acylaminoamide tels que définis dans ce qui suit, induit une augmentation de TGk dans la peau.

La TGk est une enzyme appartenant à la famille des transpeptidases. C'est une enzyme calcium-dépendante qui catalyse la formation des ponts isopeptidiques ε(γ-glutamyl) lysine entre protéines épidermiques et favorise donc la formation des enveloppes cornées.

L'association selon l'invention permet donc une amélioration de la différenciation des kératinocytes, et de la maturation et de la cohésion de la couche cornée.

Selon un autre aspect, l'invention concerne l'utilisation cosmétique d'une association d'au moins un dérivé C-glycoside avec au moins un dérivé N-acylaminoamide tels que définis en revendication 1, ou d'une composition de l'invention, pour améliorer la fonction barrière de la peau.

Une altération de la barrière cutanée peut se produire en présence d'agressions externes de type agents irritants (détergents, acides, bases, oxydants, réducteurs, solvants concentrés, gaz ou fumées toxiques), sollicitations mécaniques (frottements, chocs, abrasion, arrachement de la surface, projection de poussières, de particules, rasage ou épilation), déséquilibres thermiques ou climatiques (froid, sécheresse, radiations), xénobiotiques (micro-organismes indésirable, allergènes) ou d'agressions internes de type stress psychologique.

Cette altération de la barrière cutanée peut notamment se traduire par un inconfort cutané, des phénomènes sensoriels et notamment des phénomènes désagréables. Les terminaisons nerveuses sensorielles de la surface de la peau ou du cuir chevelu, notamment les fibres C des neurones, les récepteurs ou capteurs chimiques, thermiques, mécaniques ou les barorécepteurs sont en effet partiellement "dénudées". L'homme peut alors éprouver une sensation d'inconfort cutané qui peut se manifester notamment par des picotements, des tiraillements, des échauffements, des démangeaisons.

Il est encore décrit l'utilisation cosmétique d'une association d'au moins un dérivé C-glycoside avec au moins un dérivé N-acylaminoamide, pour renforcer la résistance de la peau aux agressions extérieures, en particulier à la pollution et aux agents polluants (tels que les particules, l'ozone, les oxydes d'azote, les oxydes de soufre et/ou les métaux lourds comme notamment le cobalt, le cadmium ou le mercure) ou aux radicaux libres, aux variations brusques de température ou d'humidité relative de l'atmosphère, au vent, à l'air conditionné, aux contraintes mécaniques, notamment à l'irritation provoquée par certains tissus rugueux ou des soins d'hygiène trop agressifs; cette utilisation peut également viser à renforcer la résistance de la peau aux stress internes, notamment aux modifications induites par des stress psychologiques ou la fatigue qui ont un retentissement sur l'état de surface de la peau.

L'association d'au moins un dérivé C-glycoside avec au moins un dérivé N-acylaminoamide selon l'invention sera ainsi particulièrement utile pour stimuler l'hydratation naturelle et l'éclat de la peau qui parait plus lumineuse et plus douce et/ou pour améliorer et/ou diminuer le micro-relief de la peau.

Il est encore décrit un procédé de traitement cosmétique pour maintenir ou favoriser l'hydratation de la peau et/ou des muqueuses, renforcer la fonction barrière de la peau et/ou des muqueuses, et favoriser la résistance de la peau et/ou des muqueuses aux agressions externes liées à l'environnement ou aux modifications provoquées par des stress intrinsèques, comprenant l'application d'une quantité efficace d'une association d'au moins un dérivé C-glycoside avec au moins un dérivé N-acylaminoamide.

L'association sera appliquée sur la partie de la peau à traiter, en particulier sur le visage, le cou ou les mains, de façon quotidienne ou pluriquotidienne. L'application sera renouvelée tous les jours pendant une période variable selon les effets souhaités, généralement de 3 à 6 semaines, mais pourra être prolongée ou poursuivie en continu.

Il est encore décrit l'utilisation cosmétique, dans une composition contenant un milieu physiologiquement acceptable, d'une association d'au moins un dérivé C-glycoside avec au moins un dérivé N-acylaminoamide, comme agent pour favoriser la différenciation et/ou la cornéification des kératinocytes; en particulier, elle vise l'utilisation de cette association comme agent pour maintenir et/ou renforcer l'épiderme, pour maintenir l'intégrité et/ou l'épaisseur des différentes couches de l'épiderme en particulier du stratum corneum, pour maintenir et/ou améliorer la fonction barrière de la peau, et/ ou pour favoriser l'hydratation naturelle de la peau.

L'utilisation de l'association est particulièrement avantageuse comme agent de soin destiné à favoriser et/ou restaurer la cohésion de la couche cornée et assurer ainsi une bonne homogénéité de la surface de la peau et des lèvres favorisant notamment ainsi un meilleur rendu esthétique des compositions de maquillage (homogénéité et tenue).

L'utilisation de cette association est également avantageuse pour la préparation de compositions pharmaceutiques destinées au traitement de pathologies cutanées liées à une cornification anormalement faible de la couche cornée.

Elle est également avantageuse dans des procédés de préparation d'épidermes et/ou de peaux reconstruites.

Il est encore décrit l'utilisation d'une association d'au moins un dérivé C-glycoside avec au moins un dérivé N-acylaminoamide, pour la préparation d'une composition dermatologique destinée à lutter contre les altérations de la fonction barrière et/ou les modifications de l'intégrité du stratum corneum.

Il est encore décrit l'utilisation d'au moins un dérivé C-glycoside en association avec un dérivé N-acylaminoamide pour la préparation d'une composition dermatologique destinée à prévenir et/ou traiter les signes de vieillissement cutané et/ou à stimuler la synthèse du collagène, en particulier du procollagène I et/ou à augmenter la différenciation des kératinocytes.

Il est encore décrit un procédé de traitement cosmétique de la peau du corps ou du visage, y compris le cuir chevelu, dans lequel on applique sur la peau une composition cosmétique telle que définie ci-dessus.

Il est encore décrit un procédé de traitement thérapeutique destiné à prévenir et/ou traiter les signes du vieillissement cutané comprenant l'administration à un sujet d'au moins un dérivé N-acylaminoamide en association avec au moins un dérivé C-glycoside.

Ainsi, les résultats figurant dans les exemples ci-après montrent qu'en associant un dérivé C-glycoside, avec un dérivé N-acétylaminoamide, on augmente la différenciation des kératinocytes. Cette augmentation conforte la stratification de l'épiderme, ce qui a pour effet de renforcer la fonction barrière de l'épiderme et donc de protéger la peau des dommages environnementaux.

### DERIVE N-ACYLAMINOAMIDE

Les composés de ce type répondent à la formule (I) suivante : 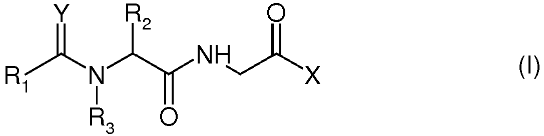 dans laquelle :
- le radical Y représente O ou S,
- le radical R₁ représente un radical méthyle, éthyle, propyle ou isopropyle, éventuellement substitué par un groupement -OH ou -P(O)-(OR)₂ avec R représentant méthyle, éthyle, propyle ou isopropyle;
- le radical R₂ représente un radical méthyle, éthyle, propyle, isopropyle, n-butyle, ter-butyle ou isobutyle;;
- le radical R₃ représente un groupement choisi parmi l'une des formules suivantes: 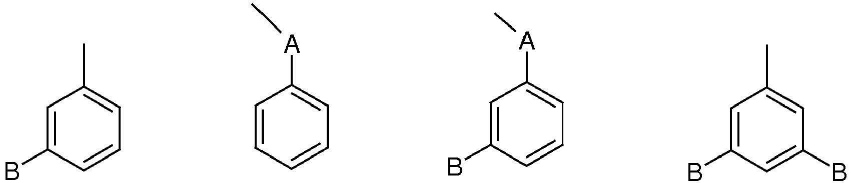 dans lesquelles le radical divalent A est un méthylène, un éthylène, un propylène et le radical B représente au moins un groupement -OR; -NHR; -CN; -COOR; - COR pour lesquels R désigne un radical méthyle, éthyle, propyle ou isopropyle, ou représente un radical hydrocarboné choisi parmi un radical méthyle, éthyle, propyle ou isopropyle, substitué par un ou plusieurs halogènes, notamment chlore, brome, iode ou fluor, et préférentiellement totalement halogéné (perhalogéné), tel que perfluoré, notamment le radical perfluorométhyle (-CF₃),
- le radical X représente un radical choisi parmi -OH, -OCH₃, -OC₂H₅, -OC₃H₇ ou -OC₄H₉;

Sont également compris dans cette définition, les sels d'acide minéral ou organique desdits composés, ainsi que leurs isomères optiques, sous forme isolée ou en mélange racémique.

De préférence, le radical Y représente l'oxygène.

Parmi les composés de formule (I), on utilise de préférence ceux pour lesquels :
- Y représente l'oxygène,
- R₁ représente un radical méthyle, éthyle, propyle ou isopropyle, éventuellement substitué par un groupement -OH ou -P(O)-(OR)₂ avec R représentant méthyle, éthyle, propyle ou isopropyle,
- R₂ représente un radical méthyle, éthyle, propyle, isopropyle, n-butyle, ter-butyle ou isobutyle,
- R₃ représente un groupement choisi parmi l'une des formules suivantes : 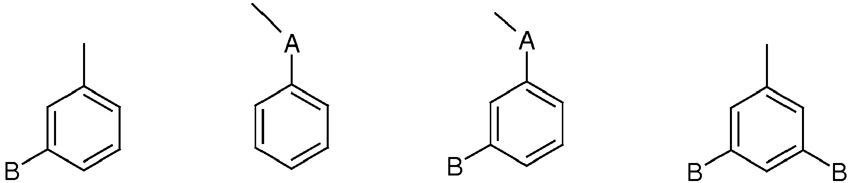 dans lesquelles
   A est un méthylène, un éthylène, un propylène et B représente au moins un groupement -OR; -NHR; -CN; -COOR; -COR pour lesquels R désigne un radical méthyle, éthyle, propyle ou isopropyle, ou représente un radical hydrocarboné choisi parmi un radical méthyle, éthyle, propyle ou isopropyle, substitué par un ou plusieurs halogènes, notamment chlore, brome, iode ou fluor, et préférentiellement totalement halogéné (perhalogéné), tel que perfluoré, notamment le radical perfluorométhyle (-CF₃), et
- X représente un radical choisi parmi -OH, -OCH₃, -OC₂H₅, -O-C₃H₇ ou -OC₄H₉.

Parmi les composés particulièrement préférés, on peut citer :
- l'acide {2-[acétyl-(3-trifluorométhyl-phényl)-amino]-3-méthyl-butyrylamino} acétique,
- le {2-[acétyl-(3-trifluorométhyl-phényl)-amino]-3-méthyl-butyrylamino} acétate d'éthyle,
- l'acide [2-(acétyl-benzyl-amino)-3-méthyl-butyrylamino] acétique,
- le [2-(acétyl-benzyl-amino)-3-méthyl-butyrylamino] acétate d'éthyle, et
- le (2-{benzyl-[(diethoxy-phosphoryl)-acétyl]-amino}-3-méthyl-butyrylamino) acétate d'éthyle.

En particulier, le composé de formule (I) selon l'invention est un pseudodipeptide constitué par l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valylglycine ou acetyl trifluoromethyl phenyl valylglycine , ou un ester de celui-ci notamment avec un alcool en C₁-C₆.

Les composés selon l'invention peuvent être aisément préparés par l'homme du métier sur base de ses connaissances générales. On peut notamment faire réagir ensemble un acide carboxylique, un aldéhyde, un composé aminé et un isonitrile, selon la réaction de Ugi.

Bien entendu, lors de la synthèse des composés selon l'invention, et en fonction de la nature des différents radicaux présents sur les composés de départ, l'homme du métier pourra veiller à protéger certains substituants pour que ceux-ci n'interviennent pas dans la suite des réactions.

La quantité en composé(s) à utiliser dans les compositions selon l'invention peut être aisément déterminée par l'homme du métier, en fonction de la nature des composés utilisés, de la personne à traiter et/ou de l'effet recherché.

D'une manière générale, cette quantité peut être comprise entre 0,00001 et 20 % en poids par rapport au poids total de la composition, notamment entre 0,001 et 10 % en poids, et de préférence entre 0,05 et 5 % en poids, encore mieux entre 0,1 et 2 % en poids, et préférentiellement entre 0,5 et 1 % en poids.

### DERIVES C-GLYCOSIDE

Le dérivé C-glycoside répond à la formule générale (IV) suivante : 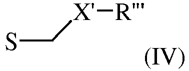 dans laquelle :
R'" représente un radical alkyle linéaire non substitué en C₁-C_{4,},
   - X' représente un groupement -CH(OH)- ;
   - S représente le D-xylose, et
   - la liaison S-CH₂-X' représente une liaison de nature C-anomérique, qui peut être α ou β,
ainsi que leurs sels cosmétiquement acceptables, leurs solvates tels que les hydrates et leurs isomères.

Dans le cadre de la présente invention, par « halogène », on entend le chlore, le fluor, le brome ou l'iode.

Parmi les groupes alkyle, on peut notamment citer les groupes méthyle, éthyle, n-propyle, n-butyle.

Préférentiellement R"' désigne un radical alkyle linéaire non substitué en C₁-C₂, en particulier éthyle.

Les sels acceptables pour l'usage non thérapeutique des composés décrits dans la présente invention comprennent des sels non toxiques conventionnels desdits composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Lorsque le composé de formule (IV) comporte un groupe acide, la neutralisation du ou des groupes acides peut être effectuée par une base minérale, telle que LiOH, NaOH, KOH, Ca(OH)₂, NH₄OH, Mg(OH)₂ ou Zn(OH)₂; ou par une base organique telle qu'une alkylamine primaire, secondaire ou tertiaire, par exemple la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine, la diméthylamino-2-propanol, le 2-amino-2-(hydroxyméthyl)-1,3-propanediol. On peut encore citer la lysine ou la 3-(diméthylamino)propylamine.

Les solvates acceptables pour les composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

Parmi les dérivés C-glycoside de formule (IV), utilisés selon l'invention, on considère tout particulièrement :
5. C-β-D-xylopyranoside-2-hydroxy-propane ;
6. C-α-D-xylopyranoside-2-hydroxy-propane ;
   - leurs isomères et leurs mélanges.

Selon un mode de réalisation, le C-β-D-xylopyranoside-2-hydroxy-propane ou le C-α-D-xylopyranoside-2-hydroxy-propane, et mieux le C-β-D-xylopyranoside-2-hydroxy-propane, peuvent être avantageusement mis en oeuvre pour la préparation d'une composition selon l'invention.

Selon un mode de réalisation particulier, le dérivé C-glycoside est le C-β-D-xylopyranoside-2-hydroxy-propane sous forme d'une solution à 30 % en poids en matière active dans un mélange eau/propylène glycol (60/40 % en poids) tel que le produit fabriqué par CHIMEX sous la dénomination commerciale « MEXORYL SBB® ».

Bien entendu, selon l'invention, un dérivé C-glycoside répondant à la formule (IV) peut être utilisé seul ou en mélange avec d'autres dérivés C-glycosides et en toute proportion.

Un dérivé C-glycoside convenant à l'invention peut notamment être obtenu par la méthode de synthèse décrite dans le document WO 02/051828.

La quantité de dérivé C-glycoside à mettre en oeuvre dans une composition selon l'invention dépend de l'effet cosmétique ou thérapeutique recherché, et peut donc varier dans une large mesure.

L'homme de l'art peut aisément, sur la base de ses connaissances générales, déterminer les quantités appropriées.

Une composition selon l'invention peut comprendre un dérivé C-glycoside à raison d'environ 0,0001 % à environ 25 % en poids en matière active par rapport au poids total de la composition, et en particulier d'environ 0,001 % à environ 10 % en poids en matière active, et plus particulièrement d'environ 0,05 % à environ 5 % en poids en matière active de dérivé C-glycoside par rapport au poids total de la composition.

Selon un mode de réalisation, une composition conforme à l'invention peut avantageusement comprendre à titre de dérivé N-acylaminoamide choisi parmi l'acide {2-[acétyl-(3-trifluorométhyl-phényl)-amino]-3-méthyl-butyrylamino} acétique, ou un de ses esters tels que par exemple le {2-[acétyl-(3-trifluorométhyl-phényl)-amino]-3-méthyl-butyrylamino} acétate d'éthyle et, à titre de dérivé C-glycoside, au moins le C-β-D-xylopyranoside-2-hydroxy-propane.

Selon une variante de l'invention le dérivé N-acylaminoamide et le dérivé C-glycoside peuvent être associés dans un rapport pondéral variant de 0,1 à 20 ; en particulier de 0,5 à 10 ; notamment de 0,8 à 2, par exemple de 1.

Les deux types de dérivés tels que définis précédemment peuvent notamment être employés, en association, dans une composition qui comprend un milieu physiologiquement acceptable, notamment dans une composition cosmétique ou dermatologique qui comprend donc par ailleurs un milieu cosmétiquement ou pharmaceutiquement acceptable.

Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.

D'une manière générale, ce milieu peut être anhydre ou aqueux. Il peut ainsi comprendre une phase aqueuse et/ou une phase grasse.

Pour une application sur la peau, la composition peut avoir la forme notamment de solution aqueuse ou huileuse; de dispersion du type lotion ou sérum; d'émulsions de consistance liquide ou semi-liquide du type lait obtenues par dispersion d'une phase grasse dans une phase aqueuse (H/E) ou inversement (E/H); de suspensions ou émulsions de consistance molle du type crème ou gel aqueux ou anhydres; de microcapsules ou microparticules; de dispersions vésiculaires de type ionique et/ou non ionique.

Pour une application sur les cheveux, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.

Ladite phase aqueuse peut comprendre en outre des alcools tels que des monoalcools en C₁-C₆ et/ou des polyols tels que le glycérol, le butylèneglycol, l'isoprène glycol, le propylèneglycol, le polyéthylèneglycol.

Lorsque la composition selon l'invention se présente sous la forme d'une émulsion, elle peut éventuellement comprendre en outre un tensioactif, de préférence en une quantité de 0,01 à 30 % en poids par rapport au poids total de la composition. La composition selon l'invention peut également comprendre au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut également comprendre une phase grasse, notamment constituée de corps gras liquides à 25°C, tels que des huiles d'origine animale, végétale, minérale ou synthétique, volatiles ou non; de corps gras solides à 25°C tels que des cires d'origine animale, végétale, minérale ou synthétique; de corps gras pâteux; de gommes; de leurs mélanges.

Les huiles volatiles sont généralement des huiles ayant, à 25°C, une tension de vapeur saturante au moins égale à 0,5 millibar (soit 50 Pa).

Parmi les constituants de la phase grasse, on peut citer :
- les silicones volatiles cycliques ayant de 3 à 8 atomes de silicium, de préférence de 4 à 6.
- les cyclocopolymères du type diméthylsiloxane/méthylalkylsiloxane,
- les silicones volatiles linéaires ayant de 2 à 9 atomes de silicium.
- les huiles volatiles hydrocarbonées, telles que les isoparaffines et notamment l'isododécane et des huiles fluorées.
- les polyalkyl(C₁-C₂₀) siloxanes et notamment ceux à groupements terminaux triméthylsilyle, parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées,
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule R₁COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.
- les gommes de silicones;
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25 °C, les ozokérites, les esters gras et les glycérides concrets à 25 °C ; les cires de polyéthylène et les cires obtenues par synthèse de Fischer-Tropsch; des huiles hydrogénées concrètes à 25 °C ; des lanolines; des esters gras concrets à 25 °C ; les cires de silicone; les cires fluorées.

De façon connue, la composition selon l'invention peut comprendre les adjuvants habituels dans le domaine considéré, tels que les gélifiants hydrophiles ou lipophiles, les additifs hydrophiles ou lipophiles, les actifs notamment cosmétiques ou pharmaceutiques hydrophiles ou lipophiles, les conservateurs, les antioxydants, les solvants, les parfums, les charges, les pigments, les nacres, les filtres UV, les absorbeurs d'odeur et les colorants. Ces adjuvants, selon leur nature, peuvent être introduits dans la phase grasse, dans la phase aqueuse et/ou dans des sphérules lipidiques.

La nature et la quantité de ces adjuvants peuvent être choisies par l'homme du métier, sur la base de ses connaissances générales, de manière à obtenir la forme de présentation désirée pour la composition. En tout état de cause, l'homme du métier veillera à choisir tous les éventuels composés complémentaires et/ou leur quantité, de manière telle que les propriétés avantageuses de la composition selon l'invention ne soient pas, ou substantiellement pas, altérées par l'adjonction envisagée.

Les compositions cosmétiques ou pharmaceutiques selon l'invention peuvent notamment se présenter sous la forme d'une composition destinée au soin et/ou au traitement des zones ulcérées ou ayant subi un stress ou microstress cutané, notamment généré par une exposition aux UV et/ou la mise en contact avec un produit irritant.

Ainsi, les compositions selon l'invention peuvent notamment se présenter sous la forme :
- d'un produit de soin, de traitement, de nettoyage ou de protection, de la peau du visage ou du corps y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées; une composition démaquillante; un lait pour le corps, notamment hydratant éventuellement après-soleil;

- d'une composition de protection solaire, de bronzage artificiel (autobronzant) ou de soin après-soleil;
- d'une composition capillaire, et notamment une crème ou un gel protecteur solaire; une composition de soin du cuir chevelu, notamment anti-chute ou repousse des cheveux ;
- d'un produit de maquillage de la peau du visage, du corps ou des lèvres, tel qu'un fond de teint, une crème teintée, un fard à joues ou à paupières, une poudre libre ou compacte, un stick anti-cernes, un stick camouflant, un rouge à lèvres, un soin des lèvres;
- Les compositions selon l'invention trouvent une application préférée comme composition de soin de la peau du visage, de type anti-rides ou anti-age, et comme composition de protection solaire ou après-soleil.

La composition utilisée selon l'invention peut en outre contenir d'autres actifs, et notamment au moins un composé choisi parmi : les agents hydratants ; les agents dépigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou stimulant la différenciation des kératinocytes ; les agents myorelaxants ; les agents tenseurs ; les agents anti-pollution et/ou anti-radicalaire, les filtres solaires, et leurs mélanges.

Par "agent hydratant", on entend :
- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ;
- soit un composé activant les glandes sébacées tel que la DHEA, ses dérivés 7-oxydés et/ou 17-alkylés, les sapogénines et la vitamine D et ses dérivés.

Les agents dépigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone, sans que cette liste soit limitative.

Par "agent anti-glycation", on entend un composé prévenant et/ou diminuant la glycation des protéines de la peau, en particulier des protéines du derme telles que le collagène.

Les agents stimulant la différenciation des kératinocytes comprennent par exemple les minéraux tels que le calcium ; l'extrait de lupin commercialisé par la société SILAB sous la dénomination commerciale Photopréventine®; le beta-sitosteryl sulfate de sodium commercialisé par la société SEPORGA sous la dénomination commerciale Phytocohésine®; et l'extrait de maïs commercialisé par la société SOLABIA sous la dénomination commerciale Phytovityl® ; et les lignanes tels que le sécoisolaricirésinol.

La composition selon l'invention peut comprendre des agents dermo-décontractants, parmi lesquels on peut citer en particulier l'alvérine et ses sels, notamment le citrate d'alvérine, les sapogénines telles que la diosgénine et les extraits naturels en contenant (tels que les extraits de Wild Yam), certaines amines secondaires et tertiaires carbonylées, des sels organiques ou inorganiques de métaux, en particulier le gluconate de manganèse, l'adénosine, ainsi que l'hexapeptide argireline R commercialisé par la société LIPOTEC. On peut également citer certaines compositions parfumantes à effet dermo-décontractant.

La présente invention décrit un procédé de traitement cosmétique de la peau du corps ou du visage, y compris le cuir chevelu, dans lequel on applique sur la peau une composition cosmétique comprenant une quantité efficace d'au moins un composé de formule (I), à laisser celle-ci en contact puis éventuellement à rincer.

Un procédé de traitement cosmétique peut être mis en oeuvre notamment en appliquant les compositions cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs; application d'une lotion pour cuir chevelu sur cheveux mouillés; application de dentifrice sur les gencives.

L'invention est illustrée plus en détail dans les exemples suivants qui sont présentés à titre illustratif et non limitatif du domaine de l'invention.

Les essais réalisés ci-après mettent en oeuvre des kératinocytes épidermiques humains normaux (NHEK) de type R3KO15 issus de plastie mammaire.

L'activité d'une composition conforme à l'invention est testée à l'égard de ces kératinocytes par dosage de l'activité transglutammase kératinocytaire (TGk) à l'issu d'un contact prolongé des kératinocytes avec la composition testée, l'augmentation de l'activité TGk sanctionnant une stimulation de la différencitation des kératinocytes.

L'activité est testée dans un milieu SFM disponible chez INVITROGEN sous la référence 17005034.

Sa composition est la suivante :
- EGF 0,25 ng/ml,
- extrait pituitaire (EP) 25 µg/ml (Invitrogen 3700015),
- Gentamycine 25 µg/ml (Sigma G1397)
supplémenté ou non en chlorure de calcium 1,5 mM final (Prolabo 22317297).

Le dérivé C-glycoside mis en oeuvre est le C-β-D-xylopyranoside-2-hydroxy-propane fabriqué par la société CHIMEX sous la dénomination commerciale « MEXORYL SBB® ». Il se présente sous la forme d'une solution à 30 % en poids en matière active dans un mélange eau/propylèneglycol 60/40.

Le dérivé N-acylaminoamide testé est l'acide {2-[acétyl-(3-trifluorométhyl-phényl)-amino]-3-méthyl-butyrylamino} acétique. Ce composé est préparé selon le protocole décrit en exemple 2 du document WO 01/94381.

Le protocole suivi pour le test d'activité est comme suit :
Les kératinocytes (R3K015) sont pré-cultivés en milieu SFM1x dans des plaques 96 puits (une plaque pour l'essai, une pour le dosage de protéines et une pour le MTT). Après incubation pendant 24 heures à 37 °C, le milieu de culture est éliminé et remplacé par du milieu SFM supplémenté ou non (contrôle sans calcium) par 1,5 mM final de chlorure de calcium et contenant ou non (témoin) les produits, mélanges à l'essai ou les références.

Après 48 heures d'incubation à 37 °C et 5 % de CO₂, le milieu de culture est éliminé et les cellules ont été à nouveau traitées puis incubées pendant 48 h à 37 °C.

Toutes les conditions expérimentales ont été réalisées en hexaplicate.

Les tapis cellulaires sont lavés puis soniqués, sur glace, en tampon Tris/EDTA, pH 8.0.

L'enzyme TGk membranaire est extraite en présence de Triton X-100 (incubation sous agitation à 4 °C), puis l'activité TGk est déterminée en mesurant l'addition covalente de putrescine tritiée à 2 µCi/ml final (Amersham TRK 414 ; 1,04 TBq/mmol, 28 Ci/mmole) à la caséine (protéine acceptrice, 2 mg/ml final), dans un tampon Tris 0,1 M, CaCl2 4 mM, EDTA 0,4 mM, DTT 5 mM, pH 8,3, pendant 2 h à 37 °C.

La caséine est précipitée par addition d'acide trichloracétique (TCA) 20 % contenant 1 mM putrescine et les précipités sont récupérés sur filtres et collecteur Skatron (lavages extensifs en TCA 5 %, 0,1 mM putrescine et éthanol) puis comptage des filtres secs en scintillation liquide.

Les protéines de chaque échantillon sont dosées à l'aide du kit Dc Protein Assay (BioRad 500-0116) et selon le protocole préconisé par le fournisseur.

La viabilité cellulaire a été évaluée par observations morphologiques et réalisation d'un test MTT.

Les résultats obtenus figurent dans le tableau 1 ci-après, l'activité TGk étant rapportée à la quantité de protéines - Milieu sans calcium.

A titre d'essai témoin, il a également été testé l'efficacité de l'acide rétinoïque.

**Tableau 1**

| Traitement | Concentration | Activité / µg | % / témoin |
|---|---|---|---|
| Témoin (-Ca) | - | 126 | 100 |
| Contrôle (+Ca) | - | 334 | 265 |
| Acide rétinoïque | 10⁻⁷ M | 111 | 88 |
| C-glycoside | 3 mM | 210 | 167 |
| | 1 mM | 171 | 136 |
| N-acylaminoamide | 0,2 mg/ml | 104 | 82 |
| | 0,04 mg/ml | 114 | 91 |
| C-glycosides + N-acylaminoamide | 3 mM + 0,2 mg/ml | 235 | 187 |
| | 1 mM + 0,04 mg/ml | 223 | 177 |

On note que :
- l'acide rétinoïque à 10⁻⁷ M provoque une réduction de l'activité TGk par rapport au témoin -Ca.

Or, dans ces conditions expérimentales, le C-glycoside testé à 3 mM et 1 mM stimule de façon significative l'activité TGk (respectivement 167 % et 136 % du témoin, p<0,01), alors que le N-acylaminoamide testé à 0,2 mg/ml et 0,04 mg/ml n'a pas d'incidence significative sur l'activité TGk.

De manière surprenante, le mélange des deux actifs aux deux concentrations testées, augmente significativement l'activité TGk présente dans les cultures de NHEK (187 % et 177 % du témoin, po<0,01).

Cette augmentation est l'effet d'une potentialisation de l'activité TGk.

## Revendications

1. Composition cosmétique et/ou dermatologique comprenant dans un milieu physiologiquement acceptable au moins un dérivé N-acylaminoamide répondant à la formule générale (I) suivante : 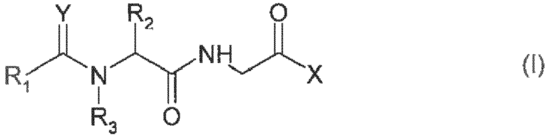 dans laquelle :
- le radical Y représente O ou S,
- le radical R₁ représente un radical méthyle, éthyle, propyle ou isopropyle, éventuellement substitué par un groupement -OH ou -P(O)-(OR)₂ avec R représentant méthyle, éthyle, propyle ou isopropyle;
- le radical R₂ représente un radical méthyle, éthyle, propyle, isopropyle, n-butyle, ter-butyle ou isobutyle;
- le radical R₃ représente un groupement choisi parmi l'une des formules suivantes: 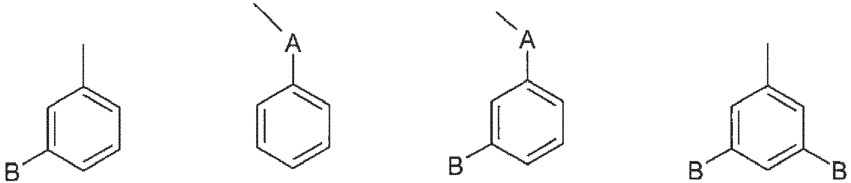 dans lesquelles le radical divalent A est un méthylène, un éthylène, un propylène et/ou le radical B représente au moins un groupement -OR; -NHR; -CN; -COOR; -COR pour lesquels R désigne un radical méthyle, éthyle, propyle ou isopropyle, ou représente un radical hydrocarboné choisi parmi un radical méthyle, éthyle, propyle ou isopropyle, substitué par un ou plusieurs halogènes, notamment chlore, brome, iode ou fluor, et préférentiellement totalement halogéné (perhalogéné), tel que perfluoré, notamment le radical perfluorométhyle (-CF₃), et,
- le radical X représente un radical choisi parmi -OH, -OCH₃, -OC₂H₅, -O-C₃H₇ ou -OC₄H₉,
et au moins un dérivé C-glycoside répondant à la formule générale (IV) suivante : 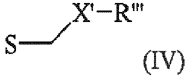 dans laquelle :
- R"' représente un radical alkyle linéaire non substitué en C₁-C₄,
- X' représente un groupement -CH(OH)-, et
- S représente le D-xylose,
la liaison S-CH₂-X' représente une liaison de nature C-anomérique, qui peut être α ou β, ainsi que leurs sels cosmétiquement acceptables, leurs solvates tels que les hydrates et leurs isomères.

2. Composition selon la revendication 1, dans laquelle ledit dérivé N-acylaminoamide est choisi parmi :
- l'acide {2-[acétyl-(3-trifluorométhyl-phényl)-amino]-3-méthyl-butyrylamino} acétique,
- le {2-[acétyl-(3-trifluorométhyl-phényl)-amino]-3-méthyl-butyrylamino} acétate d'éthyle,
- l'acide [2-(acétyl-benzyl-amino)-3-méthyl-butyrylamino] acétique,
- le [2-(acétyl-benzyl-amino)-3-méthyl-butyrylamino] acétate d'éthyle, et
- le (2- {benzyl-[(diethoxy-phosphoryl)-acétyl] -amino } -3 -méthyl-butyrylamino) acétate d'éthyle.

3. Composition selon la revendication précédente, dans laquelle ledit dérivé N-acylaminoamide est l'acide {2-[acétyl-(3-trifluorométhyl-phényl)-amino]-3-méthyl-butyrylamino} acétique ou un de ses esters.

4. Composition selon l'une quelconque des revendications 1 à 3, comprenant de 0,00001 à 20 % en poids, notamment de 0,001 à 10 % en poids, de préférence de 0,05 à 5 % en poids, en particulier de 0,1 à 2 % en poids, plus particulièrement de 0,5 à 1 % en poids de dérivé(s) N-acylaminoamide par rapport au poids total de la composition.

5. Composition selon l'une quelconque des revendications précédentes, dans laquelle R"' désigne un radical alkyle linéaire non substitué en C₁-C₂, notamment éthyle.

6. Composition selon l'une quelconque des revendications précédentes dans laquelle le dérivé C-glycoside est choisi parmi le C-β-D-xylopyranoside-2-hydroxy-propane et le C-α-D-xylopyranoside-2-hydroxy-propane, et est plus particulièrement le C-β-D-xylopyranoside-2-hydroxy-propane.

7. Composition selon l'une quelconque des revendications précédentes associant le C-β-D-xylopyranoside-2-hydroxy-propane à l'acide {2-[acétyl-(3-trifluorométhyl-phényl)-amino]-3-méthyl-butyrylamino} acétique ou un de ses esters.

8. Composition selon l'une quelconque des revendications précédentes, comprenant ledit dérivé N-acylaminoamide et ledit dérivé C-glycoside dans un rapport pondéral variant de 0,1 à 20 ; en particulier de 0,5 à 10 ; notamment de 0,8 à 2, par exemple de 1.

9. Composition selon l'une quelconque des revendications précédentes, se présentant sous la forme :
- d'un produit de soin, de traitement, de nettoyage ou de protection, de la peau du visage ou du corps y compris le cuir chevelu, tel qu'une composition de soin (de jour, de nuit, hydratante) du visage ou du corps; une composition anti-rides ou anti-âge pour le visage; une composition matifiante pour le visage; une composition pour les peaux irritées;
- d'une composition de protection solaire, de bronzage artificiel (autobronzant) ou de soin après-soleil ;
- d'une composition capillaire, et notamment une crème ou un gel protecteur solaire; une composition de soin du cuir chevelu, notamment anti-chute ou repousse des cheveux;
- d'un produit de maquillage de la peau du visage, du corps ou des lèvres, tel qu'un fond de teint, une crème teintée, un fard à joues ou à paupières, une poudre libre ou compacte, un stick anti-cernes, un stick camouflant, un rouge à lèvres, un soin des lèvres.

10. Composition selon l'une des revendications précédentes, se présentant sous la forme d'une composition de soin de la peau du visage, de type anti-rides ou anti-âge, ou d'une composition de protection solaire ou après-soleil.

11. Utilisation cosmétique d'une association d'au moins un dérivé C-glycoside tel que défini en revendications 1, 5 ou 6 avec au moins un dérivé N-acylaminoamide tel que défini en revendications 1 à 4, ou d'une composition telle que définie selon l'une des revendications 1 à 10 pour améliorer la fonction barrière de la peau.

12. Utilisation cosmétique d'au moins un dérivé N-acylaminoamide tel que défini en revendications 1 à 4, en association avec au moins un dérivé C-glycoside tel que défini en revendications 1, 5 ou 6, ou d'une composition telle que définie selon l'une des revendications 1 à 10, pour traiter, de manière préventive ou curative, les signes du vieillissement de la peau du corps ou du visage.

13. Utilisation cosmétique d'au moins un dérivé N-acylaminoamide tel que défini dans l'une des revendications 1 à 4, en association avec au moins un dérivé C-glycoside tel que défini en revendication 1, 5 ou 6, ou d'une composition telle que définie dans l'une des revendications 1 à 10, pour traiter, de manière préventive ou curative, le manque d'élasticité et/ou de tonus de la peau.

## Patentansprüche

1. Kosmetische und/oder dermatologische Zusammensetzung, umfassend, in einem physiologisch verträglichen Medium, mindestens ein N-Acylaminoamidderivat entsprechend der folgenden allgemeinen Formel (I): 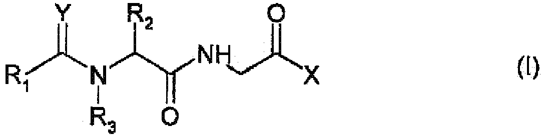 wobei:
- der Rest Y O oder S darstellt,
- der Rest R₁ einen Methyl-, Ethyl-, Propyl- oder Isopropylrest darstellt, der gegebenenfalls durch eine -OH- oder -P(O)-(O)₂-Gruppe substituiert ist, wobei R Methyl, Ethyl, Propyl oder Isopropyl darstellt:
- der Rest R₂ einen Methyl-, Ethyl-, Propyl-, Isopropyl-, n-Butyl-, tert-Butyl- oder Isobutylrest darstellt;
- der Rest R₃ eine Gruppe darstellt, die aus einer der folgenden Formeln ausgewählt ist: 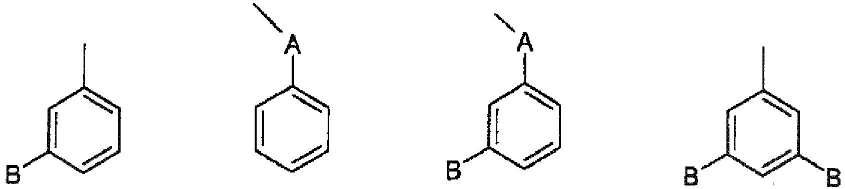 wobei der zweiwertige Rest A ein Methylen, Ethylen, Propylen darstellt und/oder der Rest B mindestens eine Gruppe -OR; -NHR; -CN; -COOR; -COR darstellt, für die R einen Methyl-, Ethyl-, Propyl- oder Isopropylrest bedeutet oder einen Kohlenwasserstoffrest darstellt, ausgewählt aus einem Methyl-, Ethyl-, Propyl- oder Isopropylrest, der durch ein oder mehrere Halogene, insbesondere Chlor, Brom, Jod oder Fluor, substituiert und vorzugsweise vollständig halogeniert (perhalogeniert), wie beispielsweise perfluoriert, insbesondere der Perfluormethylrest (-CF₃) ist, und;
- der Rest X einen Rest darstellt, ausgewählt aus -OH, -OCH₃, -OC₂H₅, -O-C₃H₇ oder - OC₄H₉,
und mindestens ein C-Glykosidderivat der folgenden allgemeinen Formel (IV): 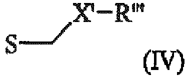 wobei:
- R'" einen unsubstituierten linearen C₁-C₄-Alkylrest darstellt,
- X' eine Gruppe -CH(OH)- darstellt, und
- S D-Xylose darstellt,
die Bindung S-CH₂-X' eine C-anomere Bindung darstellt, die α oder ß sein kann, sowie deren kosmetisch verträglichen Salze, Solvate wie Hydrate und Isomere.

2. Zusammensetzung nach Anspruch 1, wobei das N-Acylaminoamidderivat ausgewählt ist aus:
- {2-[Acetyl-(3-trifluormethyl-phenyl)-amino]-3-methyl-butyrylamino}-essigsäure,
- {2- [Acetyl-(3-trifluormethyl-phenyl)-amino] -3-methyl-butyrylamino} -ethylacetat,
- [2-(Acetyl-benzyl-amino)-3-methyl-butyrylamino]-essigsäure,
- [2-(Acetyl-benzyl-amino)-3-methyl-butyrylamino] -ethylacetat und
- (2-{Benzyl-[(diethoxy-phosphoryl)-acetyl]-amino}-3-methyl-butyrylamino)-ethylacetat.

3. Zusammensetzung nach dem vorangehenden Anspruch, wobei das N-Acylaminoamidderivat {2- [Acetyl-(3-trifluormethyl-phenyl)-amino] -3-methyl-butyrylamino}-essigsäure oder ein Ester davon ist.

4. Zusammensetzung nach einem der Ansprüche 1 bis 3, umfassend von 0,00001 bis 20 Gew.- %, insbesondere von 0,001 bis 10 Gew.-%, vorzugsweise von 0,05 bis 5 Gew.-%, insbesondere von 0,1 bis 2 Gew.-%, insbesondere von 0,5 bis 1 Gew.-% N-Acylaminoamidderivat(e) bezogen auf das Gesamtgewicht der Zusammensetzung.

5. Zusammensetzung nach einem der vorangehenden Ansprüche, wobei R'" einen linearen unsubstituierten C₁-C₂-Alkylrest, insbesondere Ethyl, bezeichnet.

6. Zusammensetzung nach einem der vorangehenden Ansprüche, wobei das C-Glykosidderivat ausgewählt ist aus C-ß-D-Xylopyranosid-2-hydroxypropan und C-α-D-Xylopyranosid-2-hydroxypropan, und insbesondere C-ß-D-Xylopyranosid-2-hydroxypropan.

7. Zusammensetzung nach einem der vorangehenden Ansprüche, die C-ß-D-Xylopyranosid-2-hydroxypropan mit {2- [Acetyl-(3-trifluormethyl-phenyl)-amino] -3-methyl-butyrylamino}-essigsäure oder einem Ester davon kombiniert.

8. Zusammensetzung nach einem der vorangehenden Ansprüche,
umfassend das N-Acylaminoamidderivat und das C-Glykosidderivat in einem Gewichtsverhältnis im Bereich von 0,1 bis 20; insbesondere von 0,5 bis 10; insbesondere von 0,8 bis 2, zum Beispiel 1.

9. Zusammensetzung nach einem der vorangehenden Ansprüche in der Form von:
- einem Produkt zur Pflege, Behandlung, Reinigung oder zum Schutz der Haut des Gesichts oder Körpers, einschließlich der Kopfhaut, wie beispielsweise eine Pflegezusammensetzung (Tag, Nacht, Feuchtigkeit) des Gesichts oder Körpers; eine Anti-Falten- oder Anti-Aging-Zusammensetzung für das Gesicht; eine mattierende Zusammensetzung für das Gesicht; eine Zusammensetzung für gereizte Haut;
- einer Zusammensetzung zum Sonnenschutz, künstlichen Bräunen (Selbstbräunen) oder Aftersun-Pflege;
- einer Haarzusammensetzung, insbesondere ein Sonnenschutzmittel oder ein Schutzgel; eine Kopfhautpflegezusammensetzung, insbesondere zur Verhinderung von Haarausfall oder zum Nachwachsen der Haare;
- einem Schminkprodukt für Gesicht, Körper oder Lippen, wie z.B. Grundierung, getönte Creme, Rouge oder Lidschatten, loser oder kompakter Puder, ein Antifaltenstift, ein Camouflage-Stift, ein Lippenstift, ein Lippenpflegestift.

10. Zusammensetzung nach einem der vorangehenden Ansprüche in Form einer Antifalten- oder Anti-Aging-Gesichtshautpflegezusammensetzung, oder einer Sonnenschutz- oder Aftersun-Zusammensetzung.

11. Kosmetische Verwendung einer Kombination aus mindestens einem C-Glykosidderivat nach Anspruch 1, 5 oder 6 mit mindestens einem N-Acylaminoamidderivat nach Anspruch 1 bis 4 oder einer Zusammensetzung nach Anspruch 1 bis 10 zur Verbesserung der Barrierefunktion der Haut.

12. Kosmetische Verwendung mindestens eines N-Acylaminoamidderivats nach den Ansprüchen 1 bis 4 in Kombination mit mindestens einem C-Glykosidderivat nach den Ansprüchen 1, 5 oder 6 oder einer Zusammensetzung nach den Ansprüchen 1 bis 10, um die Anzeichen von Hautalterung des Körpers oder des Gesichts präventiv oder kurativ zu behandeln.

13. Kosmetische Verwendung mindestens eines N-Acylaminoamidderivats nach einem der Ansprüche 1 bis 4 in Kombination mit mindestens einem C-Glykosidderivat nach Anspruch 1,5 oder 6 oder einer Zusammensetzung nach einem der Ansprüche 1 bis 10, um den Mangel an Elastizität und/oder Tonus der Haut präventiv oder kurativ zu behandeln,

## Claims

1. Cosmetic and/or dermatological composition comprising, in a physiologically acceptable medium, at least one N-acylaminoamide derivative corresponding to the following general formula (I): 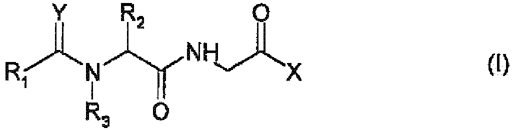 wherein:
- the radical Y represents O or S,
- the radical R₁ represents a methyl, ethyl, propyl or isopropyl radical, optionally substituted by a -OH or -P(O)-(OR)₂ group where R represents methyl, ethyl, propyl or isopropyl;
- the radical R₂ represents a methyl, ethyl, propyl, isopropyl, n-butyl, tert-butyl or isobutyl radical;
- the radical R₃ represents a group chosen from among one of the following formulas: 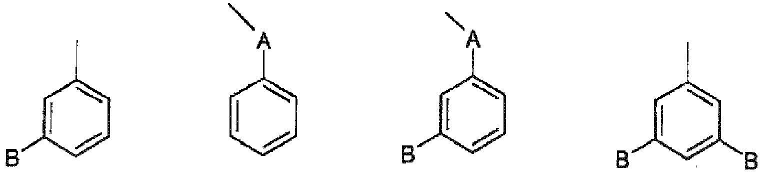 wherein the divalent radical A is methylene, ethylene, propylene and/or the radical B represents at least one -OR; -NHR; -CN; -COOR; -COR group, where R denotes a methyl, ethyl, propyl or isopropyl radical, or represents a hydrocarbon radical chosen from among a methyl, ethyl, propyl or isopropyl radical, substituted by one or more halogens, in particular chlorine, bromine, iodine or fluorine, and preferably totally halogenated (perhalogenated), such as perfluorinated, in particular the perfluoromethyl radical (-CF₃), and,
- the radical X represents a radical chosen from
among -OH, -OCH₃, -OC₂H₅, -O-C₃H₇ or -OC₄H₉,
and at least one C-glycoside derivative corresponding to the following general formula (IV): 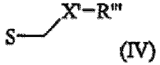 wherein:
- R"' represents an unsubstituted linear C₁-C₄ alkyl radical,
- X' represents a -CH(OH)- group, and
- S represents D-xylose,
and wherein the S-CH₂-X' bond represents a C-anomeric bond, which may be α or β, as well as their cosmetically acceptable salts, their solvates such as hydrates, and their isomers.

2. Composition according to claim 1, wherein the N-acylaminoamide derivative is selected from among:
- {2-[acetyl-(3-trifluoromethyl-phenyl)-amino]-3-methyl-butyrylamino}-acetic acid,
- {2-[acetyl-(3-trifluoromethyl-phenyl)-amino]-3-methyl-butyrylamino} ethyl acetate,
- [2-(acetyl-benzylamino)-3-methyl-butyrylamino] acetic acid,
- [2-(acetyl-benzyl-amino)-3-methyl-butyrylamino] ethyl acetate, and
- (2-{benzyl-[(diethoxy-phosphoryl)-acetyl]-amino}-3-methyl-butyrylamino) ethyl acetate.

3. Composition according to the preceding claim, wherein the N-acylaminoamide derivative is {2-[acetyl-(3-trifluoromethyl-phenyl)-amino]-3-methyl-butyrylamino} acetic acid or an ester thereof.

4. Composition according to any one of the claims 1 to 3, comprising from 0.00001 to 20% by weight, in particular from 0.001 to 10% by weight, preferably from 0.05 to 5% by weight, in particular from 0.1 to 2% by weight, more particularly from 0.5 to 1% by weight of N - acylaminoamide derivative(s) relative to the total weight of the composition.

5. Composition according to any one of the preceding claims, wherein R"' denotes an unsubstituted C₁-C₂ linear alkyl radical, in particular ethyl.

6. Composition according to any one of the preceding claims wherein the C-glycoside derivative is selected from C-β-D-xylopyranoside-2-hydroxy-propane and C-α-D-xylopyranoside-2-hydroxy-propane, and is more particularly C-β-D-xylopyranoside-2-hydroxypropane.

7. Composition according to any one of the preceding claims associating C-β-D-xylopyranoside-2-hydroxy-propane with {2-[acetyl-(3-trifluoromethyl-phenyl)-amino]-3-methyl-butyrylamino} acetic acid or one of its esters.

8. Composition according to any one of the preceding claims, comprising the N-acylaminoamide derivative and the C-glycoside derivative in a weight ratio ranging from 0.1 to 20; in particular from 0.5 to 10; in particular from 0.8 to 2, for example 1.

9. Composition according to any one of the preceding claims, in the form of:
- a product for the care, treatment, cleaning or protection of the skin of the face or body including the scalp, such as a care composition (day, night, hydrating) for the face or the body; an anti-wrinkle or anti-aging composition for the face; a mattifying composition for the face; a composition for irritated skin;
- a composition for sun protection, artificial tanning (self-tanning) or after-sun care;
- a hair composition and in particular a cream or a protective sun gel; a care composition for the scalp, in particular anti-hair loss or regrowth;
- a makeup product for the skin of the face, body or lips, such as a foundation, a tinted cream, a blush or an eyeshadow, a loose or compact powder, a stick and a concealer, a camouflaging stick, a lipstick, a lip care.

10. Composition according to one of the preceding claims, in the form of a facial skin care composition, anti-wrinkle or anti-aging, or a sunscreen composition or after-sun care product.

11. Cosmetic use of a combination of at least one C-glycoside derivative as defined in claims 1, 5 or 6 with at least one N-acylaminoamide derivative as defined in claims 1 to 4, or a composition as defined according to one of the claims 1 to 10 for improving the barrier function of the skin.

12. Cosmetic use of at least one N-acylaminoamide derivative as defined in claims 1 to 4, in combination with at least one C-glycoside derivative as defined in claims 1, 5 or 6, or a composition such as defined in one of the claims 1 to 10 for treating, in a preventive or curative manner, the signs of aging of the skin of the body or face.

13. Cosmetic use of at least one N-acylaminoamide derivative as defined in one of the claims 1 to 4, in combination with at least one C-glycoside derivative as defined in claim 1, 5 or 6, or a composition as defined in one of the claims 1 to 10, for treating, in a preventive or curative manner, the lack of elasticity and/or tone of the skin.
